# FASCICULE DE BREVET EUROPEEN

(11) **EP 1 100 401 B1**
(45) Date de publication et mention de la délivrance du brevet: **19.11.2008**
(21) Numéro de dépôt: 98941510.4
(22) Date de dépôt: 31.07.1998
(51) Int. Cl.: A61F 2/00

(54) **PROTHESE POUR LE TRAITEMENT CHIRURGICAL DES HERNIES**
PROTHESE ZUR CHIRURGISCHEN BEHANDLUNG DER HERNIA
PROSTHESIS FOR SURGICAL TREATMENT OF HERNIA

(43) Date de publication de la demande: 23.05.2001
(73) Titulaire: C.R. BARD, INC., Murray Hill New Jersey 07974 (US)
(72) Inventeur: Pelissier, Edouard, F-25870 Devecey (FR)
(74) Mandataire: Rhein, Alain
(86) Numéro de dépôt international: PCT/FR1998/001710
(87) Numéro de publication internationale: WO 2000/007520

(56) Documents cités:
- EP-A- 0 827 724
- FR-A- 2 719 993
- US-A- 5 368 602
- US-A- 5 697 978
- US-A- 5 766 246

## Description

La présente invention a pour objet une prothèse pour le traitement chirurgical des hernies.

La hernie est un défect au niveau de la paroi abdominale par lequel s'engagent le péritoine et les viscères intra-abdominaux. Elle siège le plus souvent au niveau de l'aine et de l'ombilic. Il existe aussi des hernies au niveau des incisions pratiquées lors d'une intervention chirurgicale dans l'abdomen, dénommées éventrations.

La réparation chirurgicale de la hernie poursuit deux buts, le premier est d'assurer la solidité de la paroi de façon définitive sans récidive, le second est de le faire avec aussi peu de désagréments que possible, notamment peu de douleurs afin de permettre une reprise rapide d'activité.

On notera qu'en ce qui concerne la hernie inguinale chez l'homme, la réparation est plus complexe que la simple fermeture d'un orifice, parce qu'il faut préserver le passage du cordon inguinal qui contient les vaisseaux du testicule et le canal déférent.

Le traitement chirurgical des hernies peut être réalisé par des sutures rapprochant les berges de l'orifice herniaire ou par la mise en place d'une prothèse constituée d'une pièce en treillis synthétique pour obturer l'orifice sans en rapprocher les bords. Avec une prothèse, l'absence de tension permet d'atténuer la douleur et de réduire le risque de récidive.

Il existe plusieurs types de prothèses, toutes faites d'un treillis souple de matériel synthétique, notamment de type dacron, polyéthylène, PTFE, etc.

Les prothèses existantes se présentent sous plusieurs formes. Les plus fréquentes ont la forme d'un rectangle ou d'un carré de tissu souple qui peut être appliqué tel quel ou découpé à la demande.

Certaines sont prédécoupées, le plus souvent de forme ovalaire adaptée à la zone de faiblesse de la hernie inguinale, avec une fente pour permettre le passage du cordon inguinal. D'autres sont moulées avec une certaine convexité adaptée à la forme de la paroi abdominale.

On connaît, également, une prothèse dite «plug» qui consiste en une sorte de bouchon de forme conique, destiné à être introduit dans l'orifice herniaire pour l'oblitérer.

La mise en place des prothèses peut être réalisée de différentes façons, en particulier par voie inguinale, rétropéritonéale ou par laparoscopie.

La voie rétropéritonéale ou procédé de Stoppa nécessite de réaliser une grande incision abdominale médiane afin d'accéder à l'espace rétropéritonéal et à la face profonde du plan musculaire. Cette technique permet, certes, d'étaler largement une prothèse souple à la face profonde de la paroi musculaire, de sorte que la poussée abdominale applique la prothèse contre la paroi au pourtour de l'orifice herniaire, ce qui confère une grande solidité. Cependant, on observera que la voie rétropéritonéale présente les inconvénients, d'une part, de nécessiter une incision délabrante et douloureuse et, d'autre part, de ne pas pouvoir être pratiquée sous anesthésie locale.

La laparoscopie, permet de placer la prothèse dans l'espace rétropéritonéal, tout en évitant de pratiquer une grande incision. Cependant, il s'avère que cette technique est difficile à mettre en oeuvre et demande une grande expertise de la part du chirurgien sans compter qu'elle ne peut pas être pratiquée sous anesthésie locale. De plus, cette technique est susceptible d'exposer le patient à des complications dont certaines peuvent être graves.

La voie inguinale consiste à inciser directement au niveau de la région inguinale puis, après dissection des éléments anatomiques, à mettre en place la prothèse, soit dans l'espace rétropéritonéal (procédé de Rives), soit à la face superficielle du plan musculo-aponévrotique (procédé de Lichtenstein).

Cette technique présente l'avantage d'être simple, facilement reproductible et faisable sous anesthésie locale. Cependant, on observera que, par cette technique, il est particulièrement difficile de mettre en place la prothèse dans l'espace rétropéritonéal, gage d'une solidité optimale. En effet, du fait de l'étroitesse de la voie, l'étalement des prothèses, qui sont actuellement souples, s'avère difficile de sorte que celles-ci ont tendance à former des plis. Or, l'absence d'étalement parfait à la face profonde de la paroi musculaire entraîne un risque d'engagement du sac péritonéal et augmente les possibilités de récidive.

Pour pallier à ces inconvénients, on a proposé différents appareils facilitant la mise en place et l'étalement des prothèses dans l'espace rétropéritonéal.

Ainsi, on connaît au travers des documents EP-0.557.964 et WO-92.06639, des appareils comportant un dispositif destiné à faciliter l'étalement de la prothèse dans l'espace rétropéritonéal. En fait, ces appareils comportent un dispositif tubulaire complété par une gaine et un poussoir, permettant d'introduire la prothèse à travers un trocart de laparoscopie et d'obtenir son déploiement à travers de ce trocart.

On observera que ces appareils sont, en fait, destinés, principalement, à la mise en place des prothèses par voie laparoscopique mais ne sont aucunement destinés à être utilisés pour la voie inguinale en chirurgie traditionnelle.

On connaît, également, par le document WO-96.09795 une prothèse constituée par deux couches superposées de mailles cerclées par une armature périphérique destinée à lui conférer une rigidité suffisante pour faciliter sa mise en place et son déploiement dans l'espace rétropéritonéal.

On remarquera que cette prothèse comporte plusieurs épaisseurs de maille en un matériau non résorbable, de type synthétique, et que la multiplication de ces épaisseurs entraîne une augmentation des risques d'intolérance par l'organisme, notamment en cas d'infection. L'armature est, elle aussi, constituée en un matériau non résorbable et se présente sous la forme d'un anneau relativement épais et rigide ne présentant aucune interruption. Cet anneau s'appuie alors sur les vaisseaux fémoraux, ce qui, à la longue, peut traumatiser ces derniers et entraîner des complications. En outre, le pourtour de cette prothèse présente des aspérités obtenues par découpage du bord libre et destinées à faciliter l'ancrage de ladite prothèse dans les tissus des patients. Ces aspérités sont, là encore, de nature à traumatiser les tissus, notamment les vaisseaux fémoraux et le canal déférent. De plus, cette prothèse plane et rigide n'épouse pas de manière appropriée la forme convexe du sac viscéral et de la paroi abdominale.

On connaît également, par le document US 5 368 602, un treillis chirurgical pour le recouvrement d'une ouverture pratiquée dans un tissu corporel, destiné à recouvrir l'ouverture et à laisser croître le tissu à l'intérieur du tissu chirurgical, et qui comprend un mince emplâtre constitué d'un treillis flexible délimité par un bord périphérique possédant la même flexibilité que celle dudit treillis. Ce treillis chirurgical est destiné à être mis en place par laparoscopie, il est ainsi apte à être roulé dans un trocart, puis déroulé après extraction dudit trocart. Pour faciliter l'enroulement et de déroulement, le treillis flexible comporte sur son bord périphérique au moins un élément semi-rigide possédant une configuration allongée, étroite et d'épaisseur supérieure à celle du treillis.

On connaît, finalement, par le document WO-97.22310, une prothèse composée d'une feuille souple associée à un dispositif structurel auto-dépliant destiné à faciliter l'étalement de la prothèse dans l'espace rétropéritonéal lors de sa mise en place à travers l'orifice inguinal ou par un trocart de laparoscopie. Ce dispositif peut adopter une forme arquée facilitant, uniquement, l'expansion et la mise en place de l'une des extrémités de la prothèse mais ne résolvant, en rien, les difficultés d'étalement de l'autre extrémité. Ce dispositif peut, encore, adopter la forme d'un anneau dont le pourtour s'appuie, obligatoirement, sur les vaisseaux fémoraux avec les risques de traumatisme de ces derniers évoqués ci-dessus. De plus, la nature non résorbable du matériau employé pour la réalisation de l'anneau de cette prothèse expose, là encore, le patient à des risques d'intolérance. Finalement, la forme plane de cette prothèse est incapable de s'adapter correctement à la convexité du sac péritonéal et des viscères que ce dernier contient. La présente invention a pour but de proposer une prothèse pour le traitement chirurgical des hernies, implantable par la voie inguinale sous anesthésie locale ou loco-régionale, et permettant de remédier aux inconvénients précités.

La prothèse objet de la présente invention se caractérise essentiellement en ce qu'elle comprend deux parties, à savoir un treillis synthétique non résorbable et un cerclage fixé au bord périphérique dudit treillis synthétique, ledit cerclage, qui est réalisé dans un matériau résorbable, est d'une flexibilité lui permettant de se déformer puis de reprendre sa forme initiale; et en ce que ledit cerclage présente une interruption destinée à être positionnée au droit des vaisseaux fémoraux.

Selon une caractéristique additionnelle du dispositif selon l'invention, l'association dudit treillis et dudit cerclage est réalisée de manière que ledit treillis conserve à l'intérieur dudit cerclage une certaine laxité lui permettant de prendre une forme convexe. Ceci permet une adéquation parfaite du treillis à la convexité du sac péritonéal et à la concavité de la face profonde de la paroi abdominale.

Selon une autre caractéristique additionnelle de la prothèse selon l'invention, au moins une branche positionnée diamétralement, est solidarisée par ses extrémités au cerclage, ladite branche, faite du même matériau que ledit cerclage, est de forme courbe et maintient le treillis dans une forme convexe.

Selon une autre caractéristique additionnelle de la prothèse selon l'invention, chacune des parties extrêmes du cerclage, de part et d'autre de l'interruption, présente, à proximité du bord extrême, une zone de moindre résistance permettant d'agrandir ladite interruption. Ceci permet de couper facilement le cerclage.

Selon une autre caractéristique additionnelle de la prothèse selon l'invention, le treillis présente au niveau de chacun des bords extrêmes du cerclage, une fente radiale, créant une languette destinée à être appliquée sur les vaisseaux fémoraux. Ceci évite, au bord libre de la prothèse, d'exercer une quelconque pression sur lesdits vaisseaux fémoraux.

Selon un mode de réalisation particulier de la prothèse selon l'invention, celle-ci est de forme ronde et comporte concentriquement au cerclage périphérique, un cerclage vide intérieurement de treillis, lié audit cerclage périphérique par l'intermédiaire de rayons, et présentant une interruption en regard de l'interruption dudit cerclage périphérique, les bords extrêmes des cerclages étant liés deux à deux par deux desdits rayons entre lesquels il n'y a pas de treillis, tandis qu'un fil est enfilé périphériquement à proximité dudit cerclage périphérique, ledit fil permettant, par une traction sur ses deux extrémités, de conformer la prothèse en un tronc de cône présentant latéralement un espace.

Selon une caractéristique additionnelle de la prothèse selon l'invention, le ou les cerclages ainsi que les éventuelles branches ou rayons sont constitués de fines baguettes de section ronde ou aplatie.

Selon une caractéristique additionnelle de la prothèse selon l'invention, le ou les cerclages ainsi que les éventuelles branches ou rayons sont réalisés en un matériau résorbable, notamment de type acide polyglycolique.

Les avantages et les caractéristiques du dispositif selon l'invention, ressortiront plus clairement de la description qui suit et qui se rapporte au dessin annexé, lequel en représente plusieurs modes de réalisation non limitatifs.

Dans le dessin annexé :
- la figure 1 représente une vue en plan d'un premier mode de réalisation de la prothèse selon l'invention.
- la figure 2 représente une vue de profil de la même prothèse.
- la figure 3 représente une vue en plan d'une variante de la même prothèse.
- la figure 4 représente une vue en perspective d'une autre variante de la même prothèse.
- la figure 5 représente une vue en plan d'un second mode de réalisation de la prothèse selon l'invention.
- la figure 6 représente une vue en perspective de la même prothèse dans sa configuration de mise en place.

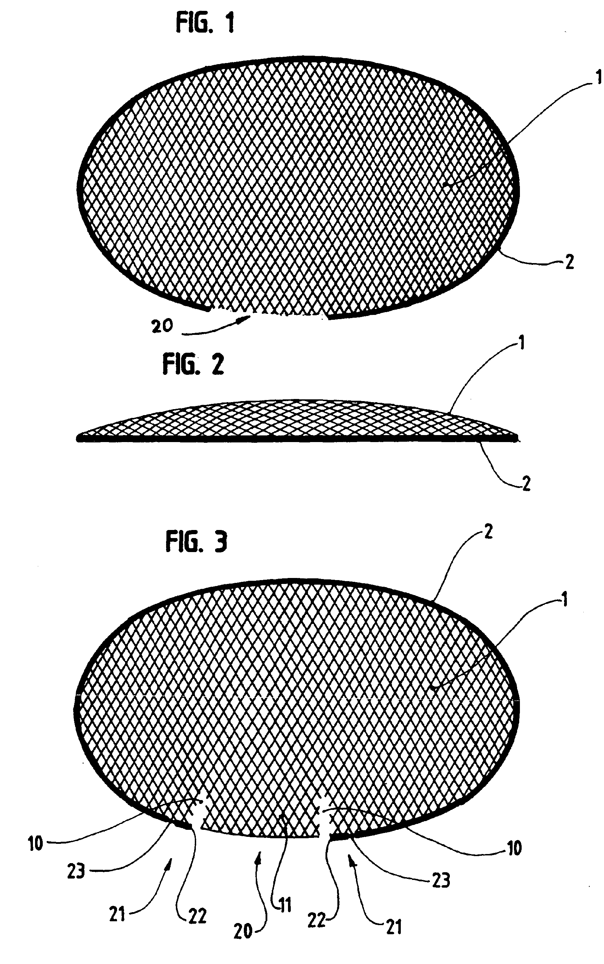

En référence aux figures 1 et 2, on peut voir que selon un premier mode de réalisation, la prothèse selon l'invention comprend un treillis 1 de forme ovalaire bordé d'un cerclage 2. Le treillis 1 est solidarisé au cerclage 2 de manière à ne pas être sous tension, c'est-à-dire à conserver une certaine laxité lui permettant de prendre une forme convexe comme cela est visible sur la figure 2.

Le treillis 1 est réalisé dans un matériau synthétique non résorbable de type polypropylène, tandis que le cerclage 2 est réalisé dans un matériau résorbable de type acide polyglycolique.

Le cerclage est destiné à conférer une mémoire de forme au treillis 1 lors de sa mise en place par la voie inguinale. Il est suffisamment souple pour être déformable sans se briser au moment de son introduction, et suffisamment rigide pour reprendre sa forme initiale et remettre le treillis 1 en tension dans l'espace rétro-péritonéal.

Le treillis 1 est ainsi complètement déployé et ne présente pas de plis, d'autant que sa forme d'origine convexe lui permet d'épouser la forme convexe du sac viscéral et la forme concave de la face profonde de la paroi abdominale. A ce propos, on observera que, selon un mode particulier de réalisation, la convexité du treillis 1 peut être conférée à ce dernier lors de sa fabrication, en particulier par moulage.

La prothèse peut être de plusieurs formes, ovalaire, ronde pour combler les pertes de subsistances grossièrement arrondies dans le cas d'un hernie ombilicale ou d'une éventration, ou piriforme c'est-à-dire sensiblement ovalaire avec une extrémité plus étroite. Elles peuvent, également, avoir des dimensions différentes pour être applicables à différents types de hernies et d'éventrations.

A ce propos et dans le cas d'une prothèse de forme ovalaire, les dimensions de cette dernière sont de 8 à 14 centimètres, de préférence 12 centimètres, pour le grand axe et de 6 à 10 centimètres, de préférence 8 centimètres, pour le petit axe.

On peut également voir sur la figure 1 que le cerclage 2 présente une interruption 20, laquelle est destinée à être positionnée en regard des vaisseaux fémoraux afin d'éviter de traumatiser ceux-ci. Dans cette conformation, le chirurgien peut fendre aux ciseaux le treillis 1 sur quelques centimètres de façon à créer une languette qui s'applique, sans tension, sur les vaisseaux fémoraux.

En référence maintenant à la figure 3, on peut voir que dans une variante, chacune des parties extrêmes 21 du cerclage 2 au niveau de l'interruption 20, présente, à proximité de l'extrémité 22 du cerclage 2, une zone 23 de moindre résistance permettant de casser le cerclage 2, de manière à agrandir l'interruption 20 si cela est nécessaire.

D'autre part, le treillis 1 présente deux fentes 10 sensiblement radiales, une au niveau de chacune des extrémités 22 du cerclage 2, qui permettent de créer une languette 11 destinée à être appliquée sur les vaisseaux fémoraux, pour éviter que ceux-ci ne soient traumatisés par le bord libre du treillis 1 qui, sans les fentes 10, serait sous tension.

On notera que la présence des fentes 10 peut être indépendante de la présence des zones de moindre résistance 23.

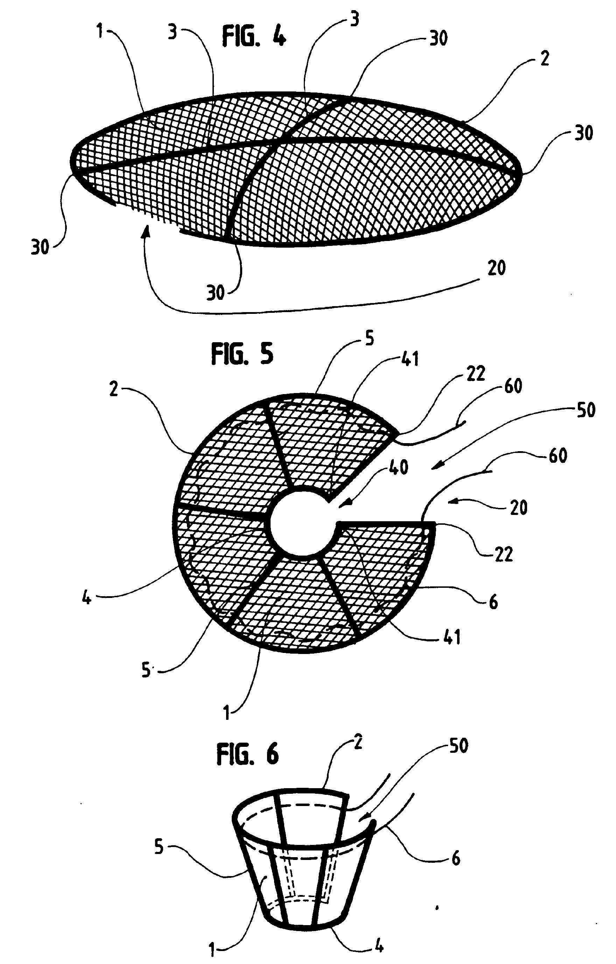

Si on se réfère maintenant à la figure 4 on peut voir que selon une variante de la prothèse selon l'invention, le cerclage 2 est associé à deux branches diamétrales 3 se croisant sensiblement à angle droit, et réalisées dans le même matériau résorbable que le cerclage 2.

Les branches 3 sont solidarisées par leurs extrémités 30 au cerclage 2 et leurs longueurs sont choisies de manière qu'elles puissent prendre une forme courbe permettant de maintenir la convexité du treillis 1.

Dans cette variante, les branches 3 sont de préférence au nombre de deux, mais il est bien entendu possible qu'une prothèse selon l'invention comporte soit une seule branche, soit plus de deux branches. Dans cette conformation, la position de l'interruption 20 du cerclage 2 doit être différente selon qu'il s'agit du coté droit ou du coté gauche.

Si on se réfère maintenant à la figure 5, on peut voir que selon un second mode de réalisation, la prothèse est de forme ronde, le cerclage 2 est doublé d'un cerclage interne 4 concentrique, vide de treillis intérieurement et lié au cerclage 2 par l'intermédiaire de rayons 5.

A ce propos, on observera qu'une telle prothèse présente des dimensions de l'ordre de 4 à 7 centimètres, de préférence 5 centimètres, pour le diamètre externe du cerclage 2 tandis que le cerclage interne 4 adopte un diamètre de 1 à 2 centimètres.

En regard de l'interruption 20 du cerclage 2, le cerclage 4 comporte une interruption 40, les bords extrêmes 22 du cerclage 2 étant liés aux bords libres 41 du cerclage 4 par deux rayons 5. On observera que les deux rayons 5 reliant les interruptions 20 et 40 délimitent un espace 50.

Selon un premier mode de réalisation représenté figure 5, entre les rayons 5 délimitant ledit espace 50, il n'y a pas de treillis. Cependant et selon un autre mode de réalisation non représenté, l'un au moins des rayons 5 délimitant ledit espace 50 est pourvu d'une languette de treillis, notamment de nature mobile. Une telle languette s'étend à l'intérieur dudit espace 50 et est destinée à se situer au droit des vaisseaux fémoraux.

Un fil 6, de préférence en un matériau résorbable, est enfilé dans le treillis 1 périphériquement à proximité du cerclage 2, ce fil permettant par une traction sur ses deux extrémités 60 qui émergent au niveau de l'interruption 20, de conformer la prothèse en un tronc de cône tel que cela est représenté sur la figure 6.

La prothèse ainsi conformée constitue une prothèse parapluie destinée au traitement des hernies inguinales indirectes.

Dans cette configuration, la prothèse peut être mise en place en étant introduite dans l'orifice inguinal, petit diamètre en avant, l'espace 50 délimité par les deux rayons 5 reliant les interruptions 20 et 40, étant destiné au passage du cordon inguinal.

Après introduction de la prothèse, le fil 6 est enlevé, ce qui permet à la prothèse de se déployer, à la manière d'un parapluie, sous l'effet élastique des cerclages 2 et 4 et des rayons 5.

Il convient, à présent, de décrire, succinctement, la technique de mise en place d'une telle prothèse.

Ainsi, il convient, après anesthésie locale ou loco-régionale, de pratiquer une incision inguinale et d'ouvrir le canal inguinal en incisant l'aponévrose. Il est ensuite pratiqué une série d'incisions et/ou de dissections adaptées à la nature, directe ou indirecte, de l'hernie traitée.

Dans le cas de la mise en place d'une prothèse telle qu'illustrée figures 1 à 4, il est, ensuite, assurée la dissection de l'espace rétro-péritonéal avant d'introduire ladite prothèse. Cette dernière est aplatie, dans le sens transversal, entre les doigts d'une main et est glissée dans la fente par sa première extrémité. La prothèse subit alors, si besoin est, une légère déformation pour assurer l'introduction de la seconde extrémité. Elle est ensuite étalée dans l'espace rétro-péritonéal, le cerclage 2 lui permettant de reprendre sa forme initiale. On ajuste le positionnement de la prothèse de sorte que les vaisseaux fémoraux se situent en regard de l'interruption 20 du cerclage 2, le treillis 1 étant, éventuellement fendu, notamment à coup de ciseaux, pour ne pas exercer de pression sur lesdits vaisseaux fémoraux. On peut alors ancrer la prothèse, notamment par suture, avant de refermer les incisions.

Si il s'agit de mettre en place une prothèse telle qu'illustrée dans les figures 5 et 6, après ouverture du canal inguinal, il est réalisé une dissection dans l'espace pré-péritonéal de manière à créer une logette destinée à accueillir la prothèse. Cette dernière est, alors, formée en tronc de cône, placée autour du bord libre du canal inguinal et introduite dans l'orifice inguinal, petit diamètre en avant. Le fil 6 est alors sectionné permettant le déploiement de la prothèse, éventuellement aidé digitalement, avant d'assurer le positionnement de cette dernière puis, si besoin est, sa fixation avant de refermer les incisions.

Il en résulte que, quel que soit le mode de réalisation de la prothèse selon l'invention, celle-ci est d'une mise en place aisée et rapide, et peut être réalisée sous anesthésie locale ou loco-régionale.

Le treillis 1 demeure toujours déployé, et s'applique parfaitement sans plis à la face profonde de la paroi muculo-aponévrotique.

## Revendications

1. Une prothèse pour le traitement chirurgical des hernies, du type comprenant deux parties, à savoir un treillis (1) constitué d'un matériau synthétique non résorbable, flexible et au moins un cerclage (2) fixé en bordure dudit treillis, **caractérisé en ce que** ledit cerclage (2), qui est réalisé dans un matériau résorbable, d'une part, s'étend de façon continue autour dudit treillis (1) pour maintenir ledit treillis dans une position déployée qui est conçue apte à recouvrir l'hernie, d'autre part, inclut des extrémités opposées (22) espacées pour former une interruption (20), et, d'autre part encore, possède une élasticité qui l'autorise à se déformer depuis une forme initiale et à revenir à la forme initiale dans laquelle ledit treillis (1) est déployé.

2. Prothèse selon la revendication 1, **caractérisée en ce que** l'association du treillis (1) et du cerclage (2) est réalisée de manière à ce que ledit treillis (1) conserve à l'intérieur dudit cerclage (2) une certaine laxité lui permettant de prendre une forme convexe.

3. Prothèse selon l'une quelconque des revendications précédentes, **caractérisée en ce qu'**au moins une branche (3) positionnée diamétralement sur ledit treillis, est solidarisée par ses extrémités (30) audit cerclage (2), ladite branche (3) est de forme courbe et maintient le treillis (1) dans une forme convexe.

4. Prothèse selon l'une quelconque des revendications précédentes, **caractérisée en ce qu'**elle comprend au moins deux branches positionnées diamétralement sur ledit treillis, et solidarisées par leurs extrémités (30) audit cerclage (2), lesdites branches sont positionnées sensiblement perpendiculaire l'une par rapport à l'autre.

5. Prothèse selon l'une quelconque des revendications précédentes, **caractérisée en ce que** chacune des parties extrêmes (21) du cerclage (2), de part et d'autre de l'interruption (20), présente, à proximité du bord extrême (22), une zone (23) de moindre résistance permettant d'agrandir ladite interruption (20).

6. Prothèse selon la revendication 5, **caractérisée en ce que** ladite zone de moindre résistance permet de casser ledit cerclage afin d'agrandir ladite interruption.

7. Prothèse selon l'une quelconque des revendications précédentes, **caractérisée en ce que** ledit treillis (1) présente deux fentes (10) au niveau de chacune des extrémités dudit cerclage (2) pour former une languette (11).

8. Prothèse selon la revendication 7, **caractérisée en ce que** ladite languette (11) est libre de toute tension.

9. Prothèse selon l'une quelconque des revendication 7 ou 8, **caractérisée en ce que** ladite languette est adaptée à être appliquée sur les vaisseaux fémoraux.

10. Prothèse selon l'une quelconque des revendications précédentes, **caractérisée en ce que** ledit cerclage (2) est doublé d'un cerclage (4) concentrique, vide de treillis intérieurement et lié audit cerclage (2) par l'intermédiaire d'au moins un rayon (5).

11. Prothèse selon la revendication 10, **caractérisée en ce que** ledit cerclage (4) comporte, en regard de l'interruption (20) dudit cerclage (2), une interruption (40), les bords extrêmes (22) du cerclage (2) étant liés aux bords (41) dudit cerclage (4) par deux rayons (5).

12. Prothèse selon la revendication 11, **caractérisée en ce que** les deux rayons délimitent un espace (50) vide de treillis.

13. Prothèses selon l'une des revendications 11 ou 12, **caractérisée en ce que** l'un au moins des rayons (5) est pourvu d'une languette de treillis, adaptée à être positionnée sur les vaisseaux fémoraux.

14. Prothèses selon l'une quelconque des revendications précédentes, **caractérisée en ce qu'**un fil (6) est enfilé dans le treillis (1) à proximité dudit cerclage (2) et permet, par une traction sur ses deux extrémités, de conformer la prothèse en un tronc de cône.

15. Prothèses selon la revendication 14, **caractérisée en ce que** ledit fil (6) est susceptible d'être enlevé dudit treillis ce qui permet à la prothèse de se déployer dans sa position initiale.

## Claims

1. A prosthesis for surgical treatment of hernias, of the type comprising two parts, namely a patch (1) made out of flexible, non-resorbable synthetic material and at least a hoop (2) fastened on the edge of said patch, **characterized in that** said hoop (2), which is made out of resorbable material, on the one hand, continuously extends around said patch (1), in order to maintain said patch in an spread out position that adapted to overlie the hernia, on the other hand, includes opposite ends (22) spaced apart so as to form an interruption (20), and, on the other hand, has an resiliency which allows its to be deformed from an initial shape and to recover the initial shape in which said patch (1) is spread out.

2. Prosthesis according to claim 1, **characterized in that** the association of the patch (1) and the hoop (2) is made so that said patch (1) keeps within said hoop (2) some flaccidity permitting its to adopt a convex shape.

3. Prosthesis according to any of the preceding claims, **characterized in that** at least one leg (3) diametrically positioned on said patch is made integral by its ends (30) to said hoop (2), said leg (3) has a curved shaped and maintains the patch (1) in a convex shape.

4. Prosthesis according to any of the preceding claims, **characterized in that** it comprises at least two legs diametrically positioned on said patch, and made integral by their ends (30) with said hoop (2), said legs are positioned substantially perpendicular with respect to each other.

5. Prosthesis according to any of the preceding claims, **characterized in that** each one of the end parts (21) of the hoop (2), on both sides of the interruption (20), has, proximate the end edge (22), an area (23) of lesser strength permitting enlarging said interruption (20).

6. Prosthesis according to claim 5, **characterized in that** said area of lesser strength permits breaking said hoop, in order to enlarge said interruption.

7. Prosthesis according to any of the preceding claims, **characterized in that** said patch (1) has two slits (10) at the level of each end of said hoop (2) in order to form a tongue (11).

8. Prosthesis according to claim 7, **characterized in that** said tongue (11) is free of any tension.

9. Prosthesis according to any of claims 7 or 8, **characterized in that** said tongue is adapted to be applied over the femoral vessels.

10. Prosthesis according to any of the preceding claims, **characterized in that** said hoop (2) is lined with a concentric wiring (4), internally without patch and connected to said hoop (2) through at least one spoke (5).

11. Prosthesis according to claim 10, **characterized in that** said wiring (4) includes, in front of the interruption (20) of said hoop (2), an interruption (40), the end edges (22) of the hoop (2) being connected to the edges (41) of said wiring (4) by means of two spokes (5).

12. Prosthesis according to claim 11, **characterized in that** the two spokes delimit a space (50) without patch.

13. Prosthesis according to any of claims 11 or 12, **characterized in that** at least one of the spokes (5) is provided with a patch tongue, adapted to be positioned over the femoral vessels.

14. Prosthesis according to any of the preceding claims, **characterized in that** a wire (6) is threaded in the patch (1) close to said hoop (2) and permits, by pulling on its two ends, shaping the prosthesis into a truncated cone.

15. Prosthesis according to claim 14, **characterized in that** said wire (6) is likely to be removed from said patch, which permits the prosthesis to spread out into its initial position.

## Patentansprüche

1. Prothese für die chirurgische Behandlung von Hernien, der Art umfassend zwei Teile, nämlich ein Flechtenwerk (1) aus einem biegsamen nicht-resorbierbaren Material und wenigstens eine am Rand des besagten Flechtenwerks befestigte Ummantelung, **dadurch gekennzeichnet, dass** die besagte Ummantelung (2), die aus einem resorbierbaren Material gefertigt ist, sich einerseits durchlaufend um das genannte Flechtenwerk (1) erstreckt, um das genannte Flechtenwerk in einer entfalteten Lage zu halten, die geeignet vorgesehen ist, um die Hernie zu bedecken, andererseits von einander beabstandete, gegenüberliegende Enden (22) umfasst, um eine Unterbrechung (20) zu bilden, und wieder andererseits eine Elastizität besitzt, die es ihr erlaubt, sich ab einer ursprünglichen Gestalt zu verformen und in die ursprüngliche Gestalt zurückzugehen, in der das besagte Flechtenwerk (1) entfaltet ist.

2. Prothese nach Anspruch 1, **dadurch gekennzeichnet, dass** die Zuordnung des Flechtenwerks (1) und der Ummantelung (2) derart durchgeführt wird, dass das besagte Flechtenwerk (1) innerhalb der besagten Ummantelung (2) eine gewisse Laxheit beibehält, die es ihm erlaubt, eine konvexe Gestalt einzunehmen.

3. Prothese nach irgendeinem der vorgehenden Ansprüche, **dadurch gekennzeichnet, dass** wenigstens ein diametral auf dem besagten Flechtenwerk positionierter Schenkel (3) mit seinen Enden (30) fest mit der besagten Ummantelung (2) verbunden ist, dass der besagte Schenkel (3) eine gebogene Gestalt hat und das Flechtenwerk (1) in einer konvexen Gestalt hält.

4. Prothese nach irgendeinem der vorgehenden Ansprüche, **dadurch gekennzeichnet, dass** sie wenigstens zwei diametral auf dem besagten Flechtenwerk positionierte und mit ihren Enden (30) fest mit der besagten Ummantelung (2) verbundene Schenkel umfasst, wobei die besagten Schenkel im wesentlichen senkrecht zu einander positioniert sind.

5. Prothese nach irgendeinem der vorgehenden Ansprüche, **dadurch gekennzeichnet, dass** jeder der äußersten Teile (21) der Ummantelung (2) beiderseits der Unterbrechung (20) in der Nähe des äußersten Randes (22) einen weniger starken Bereich (23) aufweist, der es erlaubt, die besagte Unterbrechung (20) zu vergrößern.

6. Prothese nach Anspruch 5, **dadurch gekennzeichnet, dass** der besagte weniger starke Bereich es erlaubt, die besagte Ummantelung zu brechen, um die besagte Unterbrechung zu vergrößern.

7. Prothese nach irgendeinem der vorgehenden Ansprüche, **dadurch gekennzeichnet, dass** das besagte Flechtenwerk (1) im Bereich jedes Endes der besagten Ummantelung (2) zwei Schlitze (10) umfasst, um ein Zünglein (11) zu bilden.

8. Prothese nach Anspruch 7, **dadurch gekennzeichnet, dass** das besagte Zünglein (11) völlig spannungsfrei ist.

9. Prothese nach irgendeinem der Ansprüche 7 oder 8, **dadurch gekennzeichnet, dass** das besagte Zünglein angepasst ist, um gegen die Oberschenkelgefäße angebracht zu werden.

10. Prothese nach irgendeinem der vorgehenden Ansprüche, **dadurch gekennzeichnet, dass** die besagte Ummantelung (2) mit einer konzentrischen Ummantelung (4) gefüttert ist, die innenseitig ohne Flechtenwerk ist und über wenigstens eine Speiche (5) mit der besagten Ummantelung (2) verbunden ist.

11. Prothese nach Anspruch 10, **dadurch gekennzeichnet, dass** die besagte Ummantelung (4) gegenüber der Unterbrechung (20) der besagten Ummantelung (2) eine Unterbrechung (40) umfasst, wobei die äußersten Ränder (22) der Ummantelung (2) über zwei Speichen (5) mit den Rändern (41) der besagten Ummantelung (4) verbunden sind.

12. Prothese nach Anspruch 11, **dadurch gekennzeichnet, dass** die zwei Speichen einen Raum (50) ohne Flechtenwerk begrenzen.

13. Prothese nach einem der Ansprüche 11 oder 12, **dadurch gekennzeichnet, dass** wenigstens eine der Speichen (5) mit einem Flechtenwerk-Zünglein versehen ist, das angepasst ist, um auf die Oberschenkelgefäße positioniert zu werden.

14. Prothese nach irgendeinem der vorgehenden Ansprüche, **dadurch gekennzeichnet, dass** ein Draht (6) in der Nähe der besagten Ummantelung (2) durch das Flechtenwerk (1) gestochen wird und er durch ein Ziehen an den beiden Enden erlaubt, die Prothese kegelstumpfartig zu gestalten.

15. Prothese nach Anspruch 14, **dadurch gekennzeichnet, dass** der besagte Draht (6) aus dem besagten Flechtenwerk herausgezogen werden kann, was erlaubt, dass sich die Prothese in ihre ursprüngliche Lage entfaltet.
